# EUROPEAN PATENT APPLICATION

(11) **EP 4 502 148 A1**
(43) Date of publication of application: **05.02.2025**
(21) Application number: 23774016.2
(22) Date of filing: 24.03.2023
(51) Int. Cl.: C12N 5/10, C12N 15/12, C12N 15/62, C12N 15/867, C07K 19/00, A61K 35/545, A61P 35/00, A61P 25/00, A61P 9/00

(54) **UNIVERSAL CELL AND METHOD FOR PREPARING SAME**

(30) Priority: 25.03.2022 CN 202210307870; 08.07.2022 CN 202210806871
(71) Applicant: Xellsmart Bio-Pharmaceutical (Suzhou) Co., Ltd., Suzhou, Jiangsu 210008 (CN)
(72) Inventor: LI, Xiang, Shanghai 201100 (CN); ZHU, Minzhe, Shanghai 201100 (CN)
(74) Representative: Jones Day
(86) International application number: PCT/CN2023/083761
(87) International publication number: WO 2023/179772

(57) **Abstract**

Disclosed is a method for preparing a universal cell. After major histocompatibility complex class I (MHC-I) and class II genes are inactivated in cells, fusion proteins comprising functional domains of CD47 and/or CD24 are overexpressed, such that the obtained human pluripotent stem cells or human cell lines can, on the basis of escaping T cell attack, further escape killing by NK cells, the effect of which is even better than that of the reported stellar target CD47. Meanwhile, the pluripotent stem cells with low immunogenicity retain their stemness and differentiation capacity.

## Description

### CROSS-REFERENCE TO RELATED APPLICATIONS

**The present invention claims the priority of the prior applications with a patent application number of** 202210307870.8 submitted to China National Intellectual Property Administration on March 25, 2022**, entitled "Universal cell and method for preparation same", and with a patent application number of** 202210806871.7 submitted to China National Intellectual Property Administration on July 8, 2022**, entitled "Universal cell and method for preparation same". The entire contents of these two prior applications are incorporated herein by reference.**

### TECHNICAL FIELD

The present invention belongs to the cross field of genetic engineering and stem cell technology, and specifically relates to a universal cell and a method for preparation the same.

### BACKGROUND

Through in vitro cell culture or induced differentiation of stem cells, healthy functional cells can be regenerated in large quantities in vitro, and diseases can be treated through allogeneic functional cell transplantation. However, immune incompatibility and immune rejection of transplanted cells remain key obstacles to their clinical application. Stem cells are a kind of "seed" cells with the ability of self-renewal and differentiation into specific functional cells. According to the differences of the extent of stem cell properties, stem cells are mainly divided into Totipotent stem cells, Pluripotent stem cells (PSCs) and Adult stem cells. Human embryonic stem cells (hESC) and induced pluripotent stem cells (iPSC) have the potential to proliferate indefinitely, self-renew, and differentiate into various types of cells, and have important application prospects in the treatment of cancers, nerve-related diseases, cardiovascular diseases and the like.

Transplantation of autologous cells can avoid the problem of immune rejection, but the cost of manufacturing autologous cells from patients is high and the preparation process cycle is long (Khera et al. , 2013), and the quality and effectiveness of cell products from individual sources are uncertain. Data show that the cells in patients' bodies are different from those in normal people, and the effectiveness of treatment may be affected.

Immunogenicity, or rejection reaction of the host immune system to allogeneic transplanted cells, can be reduced through immunosuppressive drugs, HLA matching, and gene editing. Immunosuppressive drugs have serious side effects and can cause bone marrow suppression, liver toxicity, hair loss and gastrointestinal adverse reactions. At present, in the United States, Japan and China, HLA-matched iPSC libraries are being established, but the construction and maintenance of the libraries are costly, because HLA antigen genes are the most polymorphic genes observed in the human genome. Currently, iPSC libraries in various regions cannot provide matches for most people in their respective countries and can only cover specific populations (Solomon et al. , 2015; Turner et al. , 2013). Allogeneic cell therapy for large patient groups may have very obvious advantages over matching libraries in terms of economy and construction and operating costs, but allogeneic cell therapy will be subject to strong immune rejection. Therefore, it is urgent to construct universal PSCs that are allogeneic immune-compatible.

The human major histocompatibility complex (MHC), i.e., the human leukocyte antigen (HLA), is the main cause of immune incompatibility. The HLA complex consists of a series of genes that can be divided into class I, class II and class III. The MHC-I gene is expressed in almost all tissue cell types, and transplanted cells expressing "non-self" MHC class I molecules will stimulate the activation of CD8+ T cells and be eliminated. CD4+ helper T cells recognize the MHC-II gene of "non-self" cells, and thus immune rejection happens, but the class III molecules do not participate in immune activities. The use of a gene editing method to modify immunogenic elements to produce cells with low immunogenicity makes it possible to manufacture "off-the-shelf" cell therapy products with immune privilege on a large scale.

In recent years, it has been previously reported that by knocking out genes such as B2M and CIITA, the expression of MHC-I and MHC-II on cell surfaces or genes themselves can be deleted, thereby making the cells have immune tolerance or escape T/B cell-specific immune responses, generating immune-compatible universal PSCs, which has laid an important foundation for the wider application of cells, tissues, and organs derived from the universal PSCs. However, HLA molecules are the major inhibitory ligands of natural killer cells (NK cells), and MHC class I-negative cells are susceptible to natural killer (NK) cell lysis. In vitro and in vivo data show that host NK cells can eliminate implanted B2M^{-/-} donor cells (Flahou et al. , 2021). Therefore, there is a need to improve previous methods to generate universal donor cells that can avoid immune responses. It has been reported that by disrupting the expression of MHC class I and MHC class II genes, cells can express non-classical HLA class I molecules such as HLA-E/G, or express immune inhibitory checkpoint proteins such as PD-L1, CTLA4-Ig, CD47, andCD24, and thus can effectively escape killing by NK cells (Zhao, W. et al. , 2020; Ye, Q. et al. , 2020;WO2021041316 A1).

These schemes have technical problems, such as incomplete, unclear or non-lasting immune compatibility, narrow effective dose window, etc., only based on direct application of common stellar molecules known in the art, thus indicating that more innovative and more optimized strategies are needed to achieve more optimized modification of stem cells to obtain better immune privilege schemes.

### SUMMARY

In response to the shortcomings of the existing technology, the present invention provides a new method for modifying cells to obtain low immunogenicity, which adopts a strategy of new genes with domain fusion for the first time, carries out a strategic practice of modifying human stem cells based on CD47 and CD24 to obtain low immunogenicity, and finally identifies a representative new fusion gene through multiple rounds of screening and sequence combination testing. The new fusion gene can significantly reduce or escape the recognition and attack of the immune system, especially the attack of natural killer cells, macrophages, etc. The present invention provides a feasible strategy to realize the immune privilege of cells by developing new genes with domain fusion.

In the present invention, by knocking out β-2-microglobulin (B2M) in the endoplasmic reticulum of human pluripotent stem cells and knocking out CIITA, a positive regulatory factor of the MHC-II gene transcription, a positive clone with B2M/CIITA double alleles knocked out (a DKO cell) is successfully constructed; then a lentivirus vector is used to overexpress a new fusion gene identified by the present invention in the DKO cell, such that the obtained human pluripotent stem cells can, on the basis of escaping T cell attack, further escape killing by NK cells, thus achieving an unexpectedly excellent immune escaping effect compared with DKO+CD47 (hereinafter referred to as "CD47 prior art"). Meanwhile, the pluripotent stem cells with low immunogenicity retain key biological functions of the pluripotent stem cells such as their stemness and differentiation capacity.

In order to achieve the above-mentioned object, the present invention adopts the following technical solutions:
In a first aspect, the present invention provides a universal cell, relative to a wild-type cell, comprising:
1) reduced expression or no expression of MHC-I and/or MHC-II human leukocyte antigens; and
2) expression of a fusion protein comprising immune inhibitory checkpoints;
wherein the cell can escape T cell attack and killing by NK cells.

In certain aspects, the fusion protein comprising immune inhibitory checkpoints include two or more of PD-L1, CTLA4-Ig, CD47, and CD24. Preferably, a fusion protein comprising two of the immune inhibitory checkpoints is expressed.

According to the present invention, a fusion protein comprising functional domains of CD47 and/or CD24 is expressed.

In certain aspects, the cell includes reduced expression or no expression of MHC-I and MHC-II human leukocyte antigens.

In certain aspects, gene editing tools (such as the TALEN and/or CRISPR system) are used in the cell to target one or more genes encoding one or more transcriptional regulatory factors of MHC-I, and one or more genes encoding one or more transcriptional regulatory factors of MHC-II, so as to achieve reduced expression or no expression of MHC-I and MHC-II genes.

In certain embodiments, in order to achieve reduced expression or no expression of MHC-I and MHC-II genes, the transcriptional regulatory factor of MHC-I can be preferably selected from one or more of B2M, TAP1, TAP2, TAP-related glycoproteins (Tapasin) or NLRC5; the transcriptional regulatory factor of MHC-II can be preferably selected from one or more of CIITA, RFXANK, RFX5 and RFXAP;
the transcriptional regulatory factors are preferably B2M and CIITA.

In certain embodiments, the cell further includes a genetic modification targeting a CIITA gene by a rare cutting endonuclease that selectively inactivates the CIITA gene.

In certain embodiments, the cell further includes a genetic modification targeting a B2M gene by a rare cutting endonuclease that selectively inactivates the B2M gene.

In certain embodiments, the genetic modification targeting the CIITA gene or the B2M gene by the rare cutting endonuclease includes a CAS protein or a polynucleotide encoding a CAS protein, and at least one guide ribonucleic acid sequence for specifically targeting the CIITA gene or the B2M.

In a specific embodiment, a CRISPR/CAS9 system is used to directly knock out the exon segments of B2M and CIITA at both ends, respectively, wherein the target sequences of gRNA for the B2M gene are SEQ ID NO: 2 and SEQ ID NO: 3, and the target sequences of gRNA for the CIITA gene are SEQ ID NO: 4 and SEQ ID NO: 5.

In certain aspects, gene expression modifying molecules for one or more genes encoding one or more transcriptional regulatory factors of MHC-I, or one or more genes encoding one or more transcriptional regulatory factors of MHC-II are introduced into the cells, so as to achieve reduced expression or no expression of MHC-I and/or MHC-II genes, wherein the gene expression modifying molecules comprise one selected from siRNA, shRNA, microRNA, antisense RNA and another RNA-mediated inhibitory molecule.

In certain aspects, the functional domain of CD47 in the fusion protein comprising the functional domains of CD47 and/or CD24 is a transmembrane domain of CD47; preferably, the amino acid sequences of the transmembrane domains of CD47 are as shown in any one of SEQ ID Nos: 6-10.

In certain aspects, the functional domain of CD24 in the fusion protein comprising the functional domains of CD47 and/or CD24 is a signal peptide sequence of CD24, a mature peptide of CD24, an extracellular peptide of CD24, a membrane anchoring sequence of CD24, and an extracellular mature peptide of CD24; preferably, the amino acid sequences of the functional domains of CD24 are as shown in any one of SEQ ID NOs: 11-16.

In certain embodiments, the fusion protein constructed by the functional domains of CD47 and CD24 is a fusion protein in which a SIRPα binding domain of CD47 is connected to the membrane anchoring sequence of CD24.

In certain embodiments, the fusion protein constructed by the functional domains of CD47 and CD24 is a fusion protein in which the SIRPα binding domain of CD47 is inserted into the linking site of the extracellular sequence and the membrane anchoring sequence of CD24.

In certain embodiments, the fusion protein constructed by the functional domains of CD47 and CD24 is a fusion protein in which the SIRPα binding domain of CD47 is linked after the extracellular sequence of CD24.

In certain embodiments, the fusion protein constructed by the functional domains of CD47 and CD24 is a fusion protein in which the mature peptide of CD24 is linked after the SIRPα binding domain of CD47.

In certain embodiments, the fusion protein constructed by the functional domains of CD47 and CD24 is a fusion protein in which the extracellular mature peptide of CD24 is inserted into the linking site of the SIRPα binding domain and the transmembrane domain of CD47.

In certain embodiments, the fusion protein constructed by the functional domains of CD47 and CD24 is a fusion protein in which the mature peptide sequence of CD24 is connected to the transmembrane domain of CD47;
preferably, the amino acid sequence of the fusion protein constructed by the functional domains of CD47 and CD24 has more than 70% homology with the sequence shown in SEQ ID NO: 1, for example more than 80% homology, and for example more than 90%, more than 95%, or more than 98% homology;
further preferably, the amino acid sequence of the fusion protein constructed by the functional domains of CD47 and CD24 is as shown in SEQ ID NO:1.

In certain aspects, the nucleic acid sequence encoding the fusion protein constructed by the functional domains of CD47 and CD24 can be obtained based on the amino acid sequence of the fusion protein.

In certain aspects, the cell further includes modifications to increase the expression of one or more of the following polypeptides: DUX4, CD27, CD35, CD46, CD55, CD59, CD200, HLA-C, HLA-E, the HLA-E heavy chain, HLA-G, PD-L1, IDO1, CTLA4-Ig, the Cl-inhibitor, IL-10, IL-35, CCL21, Mfge8 and SerpinB9.

In certain aspects, the cell is an embryonic stem cell.

In certain aspects, the cell is a pluripotent stem cell.

In certain embodiments, the cell is a stem cell with low immunogenicity.

In certain embodiments, the cell is a human stem cell or a human somatic cell.

In a second aspect, the present invention provides a method for preparing the universal cell of the first aspect, comprising the following steps:
1) knocking out one or more genes of one or more transcriptional regulatory factors of MHC-I of cells; and/or
2) knocking out one or more genes of one or more transcriptional regulatory factors of MHC-II of the cells; and
3) introducing a nucleic acid sequence encoding a fusion protein comprising immune inhibitory checkpoints into the cells.

In certain aspects, the fusion protein comprising immune inhibitory checkpoints includes more of PD-L1, CTLA4-Ig, CD47, and CD24. Preferably, a nucleic acid sequence encoding a fusion protein comprising two of the immune inhibitory checkpoints is introduced.

According to the present invention, a nucleic acid sequence encoding a fusion protein constructed by comprising functional domains of CD47 and/or CD24 is introduced.

In certain aspects, the transcriptional regulatory factor of MHC-I can be preferably selected from one or more of B2M, TAP1, TAP2, TAP-related glycoproteins (Tapasin) or NLRC5; the transcriptional regulatory factor of MHC-II can be preferably selected from one or more of CIITA, RFXANK, RFX5 and RFXAP.

In certain embodiments, the transcriptional regulatory factors are selected from B2M and CIITA.

In certain aspects, the knocking out in steps 1) and 2) is a genetic modification targeting a CIITA gene or a B2M gene by a rare cutting endonuclease that selectively inactivates the CIITA gene or the B2M gene.

Preferably, the rare cutting endonuclease is selected from CAS proteins, the TALE-nucleases, zinc finger nucleases, large nucleases and homing nucleases.

Further preferably, the genetic modification targeting the CIITA gene or the B2M gene by the rare cutting endonuclease comprises a CAS protein or a polynucleotide encoding a CAS protein, and at least one guide ribonucleic acid sequence for specifically targeting the CIITA gene or the B2M.

In certain embodiments, a CRISPR system is used in steps 1) and 2) to directly knock out the exon segments of B2M and CIITA at both ends, respectively, wherein the target sequences of gRNA for the B2M gene are SEQ ID NO: 2 and 3, and the target sequences of gRNA for the CIITA gene are SEQ ID NO: 4 and 5.

In certain aspects, the knocking out in step 1) or 2) is introducing gene expression modifying molecules for one or more genes encoding one or more transcriptional regulatory factors of MHC-I, or for one or more genes encoding one or more transcriptional regulatory factors of MHC-II, so as to achieve reduced expression or no expression of MHC-I and/or MHC-II genes, wherein the gene expression modifying molecules comprise one selected from siRNA, shRNA, microRNA, antisense RNA and another RNA-mediated inhibitory molecule.

In certain aspects, in step 3), an expression vector is used to introduce a nucleic acid sequence encoding a fusion protein comprising functional domains of CD47 and/or CD24 into cells.

Preferably, the expression vector used in the step 3) is a viral vector.

In certain embodiments, the viral vector used in the step 3) is a lentivirus.

In certain aspects, step3) introduces a nucleic acid sequence encoding a fusion protein comprising functional domains ofCD47and/orCD24into a selected site of the cell; preferably, the selected site of the cell is a safe harbor gene site.

In certain aspects, the functional domain of CD47 in the fusion protein comprising the functional domains of CD47 and/or CD24 is a transmembrane domain of CD47; preferably, the amino acid sequences of the transmembrane domains of CD47 are as shown in any one of SEQ ID Nos: 6-10.

In certain aspects, the functional domain of CD24 in the fusion protein comprising the functional domains of CD47 and/or CD24 is a signal peptide sequence of CD24, a mature peptide of CD24, an extracellular peptide of CD24, a membrane anchoring sequence of CD24, and an extracellular mature peptide of CD24; preferably, the amino acid sequences of the functional domains of CD24 are as shown in any one of SEQ ID NOs: 11-16.

In certain embodiments, the fusion protein constructed by the functional domains of CD47 and CD24 is a fusion protein in which a SIRPα binding domain of CD47 is connected to the membrane anchoring sequence of CD24.

In certain embodiments, the fusion protein constructed by the functional domains of CD47 and CD24 is a fusion protein in which the SIRPα binding domain of CD47 is inserted into the linking site of the extracellular sequence and the membrane anchoring sequence of CD24.

In certain embodiments, the fusion protein constructed by the functional domains of CD47 and CD24 is a fusion protein in which the SIRPα binding domain of CD47 is linked after the extracellular sequence of CD24.

In certain embodiments, the fusion protein constructed by the functional domains of CD47 and CD24 is a fusion protein in which the mature peptide of CD24 is linked after the SIRPα binding domain of CD47.

In certain embodiments, the fusion protein constructed by the functional domains of CD47 and CD24 is a fusion protein in which the extracellular mature peptide of CD24 is inserted into the linking site of the SIRPα binding domain and the transmembrane domain of CD47.

In certain embodiments, the fusion protein constructed by the functional domains of CD47 and CD24 is a fusion protein in which the mature peptide sequence of CD24 is connected to the transmembrane domain of CD47;

Preferably, the fusion protein constructed by the functional domains of CD47 and CD24 has more than at least 70% homology with the sequence shown in SEQ ID NO: 1, for example more than 80% homology, and for example more than 90%, more than 95%, or more than 98% homology; further preferably, the amino acid sequence of the fusion protein constructed by the functional domains of CD47 and CD24 is as shown in SEQ ID NO:1.

In certain aspects, the universal cell further comprises a second expression vector, comprising a polynucleotide sequence encoding one selected from CD35, CD27, DUX4, CD26, CD200, HLA-C, HLA-E, the HLA-E heavy chain, HLA-G, PD-L1, IDO1, IDO2, TDO, CTLA4-IgG, the Cl-inhibitor, IL-10, CD46, CD55, CD59, CCL21, Mfge8, SerpinB9 and IL-35.

In certain embodiments, the second expression vector is an inducible expression vector; preferably, the second expression vector is a viral vector.

In a third aspect, the present invention provides a method for preparing differentiated universal cells, including culturing the universal cells prepared according to the method according to the second aspect under differentiation conditions, thereby preparing differentiated cells with low immunogenicity.

In certain aspects, the differentiation conditions are suitable for differentiating cells into a cell type selected from cardiomyocytes, neurocytes, glial cells, endothelial cells, T cells, NK cells, NKT cells, macrophages, hematopoietic progenitor cells, mesenchymal cells, pancreatic islet cells, chondrocytes, retinal pigment epithelial cells, kidney cells, hepatocytes, thyroid cells, skin cells, blood cells and epithelial cells.

In a fourth aspect, the present invention provides a method for treating a patient in need of cell therapy, including administering the differentiated cell population with low immunogenicity prepared according to the method according to the third aspect.

In a fifth aspect, the present invention provides a composition, comprising the universal cell described in the first aspect.

In certain aspects, the composition comprises the universal cell described in the first aspect and one or more therapeutic agents, and the therapeutic agent comprises peptides, cytokines, small molecular compounds, macromolecules, ADC, antibodies, nanoparticles, biological analogues, mRNA, Chinese herbologies, proteins, vaccines, checkpoint inhibitors, mitogens, growth factors, small RNAs, double stranded RNAs (dsRNAs), mononuclear blood cells, feeder cells, feeder cell components or replacement factors thereof, vectors comprising one or more polynucleic acids of interest, antibodies, etc.

In a sixth aspect, the present invention provides a fusion protein, comprising the functional domains of CD47 and/or CD24.

In certain aspects, the functional domain of CD47 in the fusion protein comprising the functional domains of CD47 and/or CD24 is a transmembrane domain of CD47; preferably, the amino acid sequences of the transmembrane domains of CD47 are as shown in any one of SEQ ID Nos: 6-10.

In certain aspects, the functional domain of CD24 in the fusion protein comprising the functional domains of CD47 and/or CD24 is a signal peptide sequence of CD24, a mature peptide of CD24, an extracellular peptide of CD24, a membrane anchoring sequence of CD24, and an extracellular mature peptide of CD24; preferably, the amino acid sequences of the functional domains of CD24 are as shown in any one of SEQ ID NOs: 11-16.

In certain embodiments, the fusion protein constructed by the functional domains of CD47 and CD24 is a fusion protein in which a SIRPα binding domain of CD47 is connected to the membrane anchoring sequence of CD24.

In certain embodiments, the fusion protein constructed by the functional domains of CD47 and CD24 is a fusion protein in which the SIRPα binding domain of CD47 is inserted into the linking site of the extracellular sequence and the membrane anchoring sequence of CD24.

In certain embodiments, the fusion protein constructed by the functional domains of CD47 and CD24 is a fusion protein in which CD47 is linked after the extracellular sequence of CD24.

In certain embodiments, the fusion protein constructed by the functional domains of CD47 and CD24 is a fusion protein in which the mature peptide of CD24 is linked after the SIRPα binding domain of CD47.

In certain embodiments, the fusion protein constructed by the functional domains of CD47 and CD24 is a fusion protein in which the extracellular mature peptide of CD24 is inserted into the linking site of the SIRPα binding domain and the transmembrane domain of CD47.

In certain embodiments, the fusion protein constructed by the functional domains of CD47 and CD24 is a fusion protein in which the mature peptide sequence of CD24 is connected to the transmembrane domain of CD47;

Preferably, the fusion protein constructed by the functional domains of CD47 and CD24 has more than at least 70% homology with the sequence shown in SEQ ID NO: 1, for example more than 80% homology, and for example more than 90%, more than 95%, or more than 98% homology; further preferably, the amino acid sequence of the fusion protein constructed by the functional domains of CD47 and CD24 is as shown in SEQ ID NO:1.

In a seventh aspect, the present invention provides a nucleic acid sequence encoding the fusion protein of the sixth aspect, wherein the nucleic acid sequence can be obtained based on the amino acid sequence of the fusion protein.

In an eighth aspect, the present invention provides an expression vector or expression cassette, comprising the nucleic acid sequence of the seventh aspect.

In a ninth aspect, the present invention provides a cell, comprising the expression of the fusion protein of the sixth aspect, and reduced expression or no expression of MHC class I and/or MHC class II human leukocyte antigens.

In a tenth aspect, the present invention provides a cell, comprising no expression of CIITA, the expression of the fusion protein of the sixth aspect, and reduced expression or no expression of MHC class I and/or MHC class II human leukocyte antigens.

In an eleventh aspect, the present invention provides a cell, comprising no expression of B2M, the expression of the fusion protein of the sixth aspect, and reduced expression or no expression of MHC class I and/or MHC class II human leukocyte antigens.

In a twelfth aspect, the present invention provides a cell, comprising no expression of CIITA and B2M, the expression of the fusion protein of the sixth aspect, and reduced expression or no expression of MHC class I and/or MHC class II human leukocyte antigens.

In a thirteenth aspect, the present invention provides a cell, comprising the expression of the fusion protein of the sixth aspect and at least one polypeptide selected from DUX4, HLA-C, HLA-E, HLA-G, PD-L1, CTLA4, the Cl-inhibitor, CD46, CD55, CD59, and IL-35, and reduced expression or no expression of MHC class I and/or MHC class II human leukocyte antigens.

In a fourteenth aspect, the present invention provides a cell, comprising no expression of CIITA, the expression of the fusion protein of the sixth aspect and at least one polypeptide selected from CD35, CD27, DUX4, CD26, CD55, CD59, CD200, HLA-C, HLA-E, the HLA-E heavy chain, HLA-G, PD-L1, IDO1, IDO2, TDO, CTLA4-IgG , the Cl-inhibitor, IL-10, CD46, CD55, CD59, CCL21, Mfge8, SerpinB9 and IL-35, and reduced expression or no expression of MHC class I and/or MHC class II human leukocyte antigens.

In a fifth aspect, the present invention provides a cell, comprising no expression of B2M, the expression of the fusion protein of the sixth aspect and at least one polypeptide selected from DUX4, HLA-C, HLA-E, HLA-G, PD-L1, CTLA4, the Cl-inhibitor, CD46, CD55, CD59, and IL-35, and reduced expression or no expression of MHC class I and/or MHC class II human leukocyte antigens.

In a sixteenth aspect, the present invention provides a cell, comprising no expression of CIITA and B2M, the expression of the fusion protein of the sixth aspect and at least one polypeptide selected from CD35, CD27, DUX4, CD26, CD55, CD59, CD200, HLA-C, HLA-E, the HLA-E heavy chain, HLA-G, PD-L1, IDO1, IDO2, TDO, CTLA4-IgG , the Cl-inhibitor, IL-10, CD46, CD55, CD59, CCL21, Mfge8, SerpinB9 and IL-35, and reduced expression or no expression of MHC class I and/or MHC class II human leukocyte antigens.

In a seventeenth aspect, the present invention provides a cell described in the ninth to sixteenth aspects above, wherein the cell is selected from stem cells, differentiated cells, pluripotent stem cells, induced pluripotent stem cells, adult stem cells, progenitor cells, somatic cells, primary T cells and chimeric antigen receptor T cells.

In an eighteenth aspect, the present invention provides use of the universal cell described in the first aspect, the composition described in the fifth aspect, or the expression vector or expression cassette described in the eighth aspect in the preparation of a product for cell therapy.

In a nineteenth aspect, the present invention provides use of the universal cell described in the first aspect, the composition described in the fifth aspect, or the expression vector or expression cassette described in the eighth aspect in the preparation of a product for organ transplantation.

In a twentieth aspect, the present invention provides use of the universal cell described in the first aspect, the composition described in the fifth aspect, or the expression vector or expression cassette described in the eighth aspect in the construction of cell libraries of universal PSCs.

In a twenty-first aspect, the present invention provides use of the universal cell described in the first aspect, the composition described in the fifth aspect, or the expression vector or expression cassette described in the eighth aspect as gene drug carriers.

### Beneficial effects of the present invention:

After major histocompatibility complex class I (MHC-I) and class II genes are inactivated in stem cells in the present invention, the fusion protein XSG006 constructed by functional domains of CD47 and CD24 is overexpressed, such that the obtained human pluripotent stem cells can, on the basis of escaping T cell attack, further escape killing by NK cells, the effect of which is even better than that of the reported positive targets CD47 and CD24. Meanwhile, the pluripotent stem cells with low immunogenicity retain their stemness and differentiation capacity. Compared with neurocytes differentiated from WT cells, neurocytes differentiated from DKO+G6 cells overexpressing the fusion protein XSG006 can effectively escape the attack of the immune system in vivo.

### BRIEF DESCRIPTION OF THE DRAWINGS

Fig. 1. The diagram showing the knocking out strategy and results of the B2M gene in the human embryonic stem cell line H1;
Fig. 2. The diagram showing the knocking out strategy and results of the CIITA gene in the human embryonic stem cell line H1;
Fig. 3. The diagram showing the expression of B2M and CIITA at RNA level in DKO cells detected by RT-qPCR in Example 1;
Fig. 4. The diagram showing the results of B2M at protein level detected by Western-blot in Example 1;
Fig. 5. The diagram showing the expression of HLA-I/II in various cells detected by a flow cytometer after stimulating wild-type H1 (WT) and DKO with INF-gamma in Example 1;
   wherein, T cells are the positive control for detecting HLA-class I/II molecules;
Fig. 6. The diagram showing the karyotype of the positive clone with B2M/CIITA double alleles knocked out (DKO) obtained in Example 1;
Fig. 7. The diagram showing the expression of the stemness genes POUSF1/NANOG/SOX2 at RNA and protein levels in WT and DKO cells detected by immunofluorescence and RT-qPCR in Example 2;
   Wherein, MSC is the negative control;
Fig. 8. The diagram showing the expression of the stemness genes SSEA-4 and Tra1-81 on the surface of WT and DKO cells detected by immunofluorescence in Example 2.
Fig. 9. Immunohistochemistry is used to show that DKO cells can form a teratoma with three germ layers: an entoderm, a mesoderm, and an ectoderm;
Fig. 10. The diagram showing the results of the verification of the immune function of DKO cells detected by RTCA in Example 2;
Fig. 11. Schematic diagram of the structure of the pGC-EF1a plasmid;
Fig. 12. The diagram showing the expression of CD47 in the DKO + CD47 cell line constructed in Example 3 detected by a flow cytometer;
Fig. 13. The diagram showing the killing results of NK cells on the DKO+CD47 cell strain overexpressing CD47, WT and DKO cells detected by RTCA in Example 3;
Fig. 14. The diagram showing the killing results of NK cells on the cell strain DKO+G1-G20 overexpressed the XSG1-XSG20 fusion protein, H1WT (the positive control) and H1 DKO cells (the negative control) detected by RTCA in Example 4.
Fig. 15. The sequence PCR identification of the genome of the constructed DKO+G6 cell strain in Example 5.
Fig. 16. The diagram showing the killing results of NK cells on the DKO+G6 cell strains overexpressing the XSG006 protein, WT and DKO cells detected by RTCA in Example 5.
Fig. 17. The diagram showing the killing results of NK cells on the DKO+G6 cell strains overexpressing the XSG006 protein, WT, DKO, and the DKO+CD24/CD47 cells overexpressing the CD24 or CD47 protein detected by RTCA in Example 6;
   A is the diagram showing the killing results; B is the summary diagram of multiple results.
Fig. 18. The diagram showing the of the killing results of NK cells on the DKO+G6 cell strains overexpressing the XSG006 protein, WT, DKO, the DKO+CD24/CD47 cells overexpressing the CD24 or CD47 protein, and the DKO+CD47+CD24 cells overexpressing the CD24 and CD47 proteins detected by RTCA in Example 7;
   A is the diagram showing the killing results; B is the summary diagram of multiple results.
Fig. 19. The diagram showing the verification results of the immune escaping effect of neurocytes differentiated from DKO+G6 in Example 8 in mice with humanized immune system;

### DETAILED DESCRIPTION

The technical solution of the present invention will be further described in detail below in conjunction with specific examples. It should be understood that the following examples are only intended to illustrate and explain the present invention by way of example only, and should not be construed as limiting the scope of protection of the present invention. All technologies achieved based on the above contents of the present invention are covered in the scope that the present invention aims to protect.

Unless otherwise specified, the raw materials and reagents used in the following examples are commercially available or can be prepared by known methods. The experimental methods in the following examples where specific conditions are not specified are generally performed according to conventional conditions such as those described in Sambrook et al., Molecular Cloning: A Laboratory Manual (New York: Cold Spring Harbor Laboratory Press, 1989), or according to the conditions recommended by the manufacturer.

Unless defined otherwise or clearly indicated by the context, all technical and scientific terms in the present disclosure have the same meaning as commonly understood by one of ordinary skill in the art to which the present disclosure belongs.

### Example 1. Construction of B2M and CIITA double knocked out cell line (DKO)

1. Cell culture reagents:

| Name of reagents | Company |
|---|---|
| mTeSR^{™} Plus | Stemcell |
| TrypLE^{™} Express enzyme (1X), phenol red free | ThermoFisher |
| DPBS, calcium-free and magnesium-free | ThermoFisher |
| Matrigel^{®} hESC-Qualified Matrix, *LDEV-Free, 5 mL | Corning^{®} |
| DMEM/F-12 with GlutaMAX^{™} additive | ThermoFisher |
| Y-27632 | Stemcell |
| TrueCut^{™} Cas9 Protein v2 | ThermoFisher |
| EasyEdit sgRNA | GenScript |

2. Methods and results:
In the present invention, the human pluripotent stem cell lines H1 (Wicell, WA01) or H9 (Wicell, WA09) were selected, and β-2- microglobulin (B2M) in the endoplasmic reticulum was knocked out by CRISPR/CAS9, so that MHC-I on the cell surface cannot form functional molecules, thereby escaping killing by allogeneic CD8⁺ T cells; the escape of killing by CD4⁺ T cell was achieved by knocking out the positive regulatory factor CIITA of MHC-II gene transcription, so as to reduce the expression of MHC class II molecules.

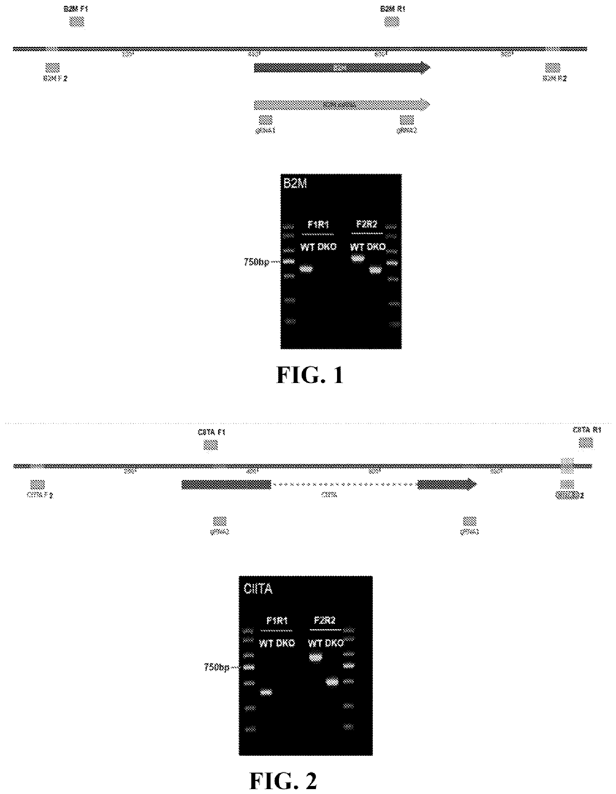

Wherein, the CRISPR/CAS9 knocking out strategy of B2M gene is shown in Fig. 1. B2M-gRNA1 and B2M-gRNA2 were used to directly knock out the exon segments of B2M at both ends, and then two pairs of PCR primers B2M-F1/R1 and B2M-F2/R2 were used for verification of genome sequence knocking out.
gRNA sequences:
   B2M-gRNA1: CGTGAGTAAACCTGAATCTT
   B2M-gRNA2: AGTCACATGGTTCACACGGC
Identification primers
   B2M-F1: TGGGGCCAAATCATGTAGACTC
   B2M-R1: TCAGTGGGGGTGAATTCAGTGT
   B2M-F2 + B2M-R2=608bp
After knocking out: no band
   B2M-F2: CAGAAGTCCTTGAGAGCCTCC
   B2M-R2: TGTGCATCAGTATCTCAGCAGG
   B2M-F2 + B2M-R2=812 bp
After knocking out: 569bp.

In addition, the CRISPR/CAS9 knocking out strategy of CIITA gene is shown in Fig. 2. CIITA-gRNA1 and CIITA-gRNA2 were used to directly knock out the exon segments of CIITA at both ends, and then two pairs of PCR primers CIITA-F1/R1 and CIITA-F2/R2 were used for verification of genome sequence knocking out.
gRNA sequences:
   CIITA-gRNA1: GATATTGGCATAAGCCTCCC
   CIITA-gRNA2: CATCGCTGTTAAGAAGCTCC
Identification primers:
   CIITA-F1: CTGTGCCTCTACCACTTCTATG
   CIITA-R1: CCTTCCATGTCACACAACAGCC
   CIITA-F1 + CIITA-R1=368 bp
After knocking out: no band
   CIITA-F2: TGGAATCCACACTTTCCAGTTC
   CIITA-R2: TGGAGTCTCCGTTCCTCCAG
   CIITA-F2 + CIITA-R2=889 bp
After knocking out: 459bp

The specific operations were as follows:
1) Human pluripotent stem cells were normally cultured to 80% density on a 6-well plate coated with Matrigel using mTeSR1. After TRYPLE digestion, DMEM/F12 was added for neutralization and counting was carried out. 2×10⁶ cells were absorbed in an EP tube, and the supernatant was discarded after centrifugation.
2) Based on the Neon transfection system, i.e., 100 µL of an electrotransfection system, 15 µg of TrueCut^{™} Cas9 Protein + 3 µg of gRNA (B2MgRNA1 + B2MgRNA2 + CIITA gRNA1 + CIITA gRNA2) were added to form an RNP system, mixed well and left at room temperature for 20 min.
3) The cells were resuspended by 100 µL of the RNP electrotransfection system, and electrotransfected by the Neon transfection system with the electrotransfection parameters of 1200 V, 30 ms and 1 pause. After electrotransfection, the cells were quickly added to a preheated medium and evenly inoculated into one of the 6-well plate coated with Matrigel.
4) The medium was replaced with a fresh mTeSR1 medium every day. When single cells grew, a single clone was picked into a 48-well plate. After the clone was amplified, genome samples were collected for PCR to detect gene editing. The PCR results are shown in Fig. 1 and 2. PCR-positive clones were sent to the company for Sanger sequencing for further verification.
5) The positive clone with B2M/CIITA double alleles knocked out DKO was identified, amplified, cultured and cryopreserved.

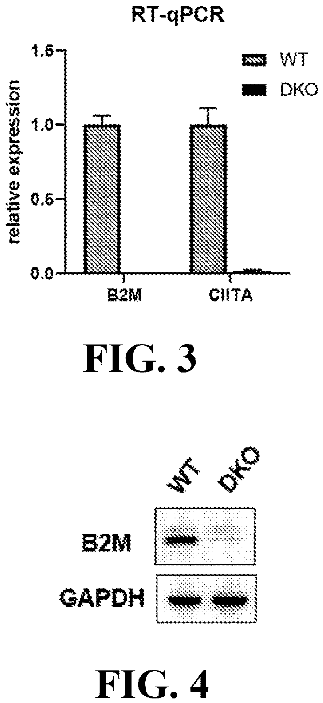

The expression of B2M and CIITA at RNA level in the clone DKO with B2M/CIITA double alleles knocked out was detected by qPCR as shown in Fig. 3 to confirm the knocking out.
B2M-F: AAGATGAGTATGCCTGCCGT
B2M-R: ATGCGGCATCTTCAAACCTC
CIITA-F: CCTGGAGCTTCTTAACAGCGA
CIITA-R: TGTGTCGGGTTCTGAGTAGAG

The expression of B2M at protein level in the clone DKO with B2M/CIITA double alleles knocked out was detected by Western-Blot as shown in Fig. 4 to confirm the knocking out.

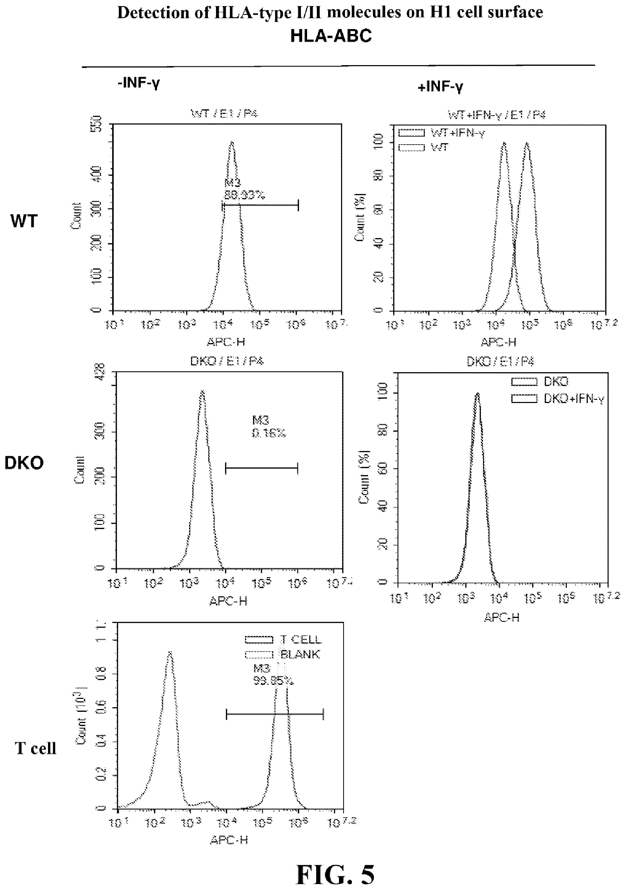

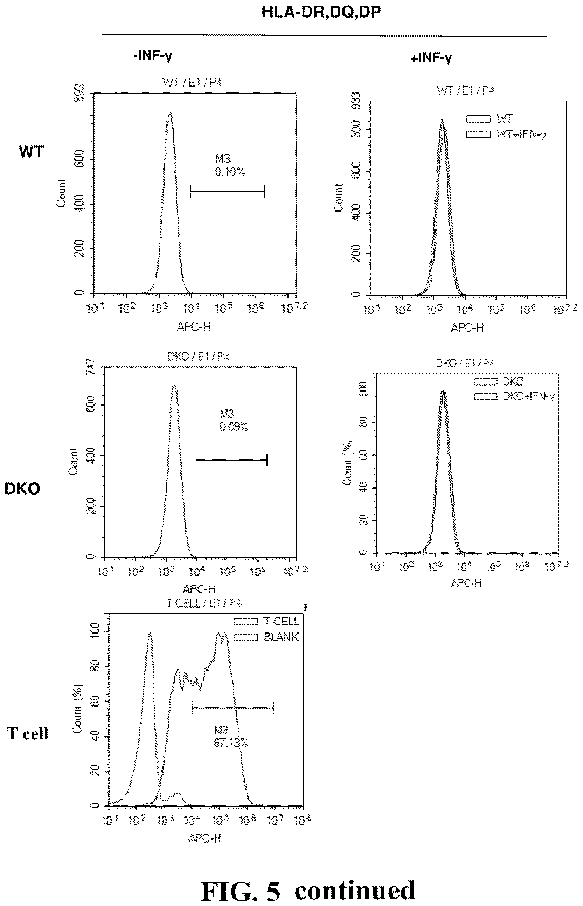

Stimulation of WT and DKO with INF-gamma: The cells were plated, and the medium containing INF-gamma was added to the cells when the liquid was changed the next day. After 48 hours, the cells were digested and the expression of HLA-I/II was detected by flow cytometry. The Results are shown in Fig. 5, the stem cell positive clones (DKO) with B2M/CIITA double alleles knocked out cannot express HLA-class I/II molecules in response to the stimulation of INF-gamma. T cells are the positive control for detecting HLA-class I/II molecules.

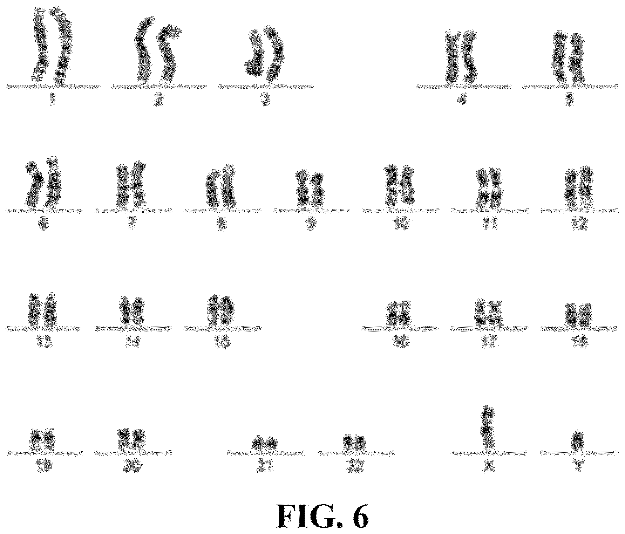

Karyotype detection of the obtained positive clones (DKO) with B2M/CIITA double alleles knocked out: the chromosome specimens fixed on the slide were treated with trypsin and then stained with Giemsa stain. The chromosome number and morphological structure of metaphase chromosomes were analyzed to determine whether their karyotypes were consistent with the normal karyotypes. The results are shown in Fig. 6, and the karyotype of DKO is normal.

### Example 2. Verification of stemness and immune function of DKO cell line in Example 1

### 1. Expression of stemness genes in WT and DKO cells

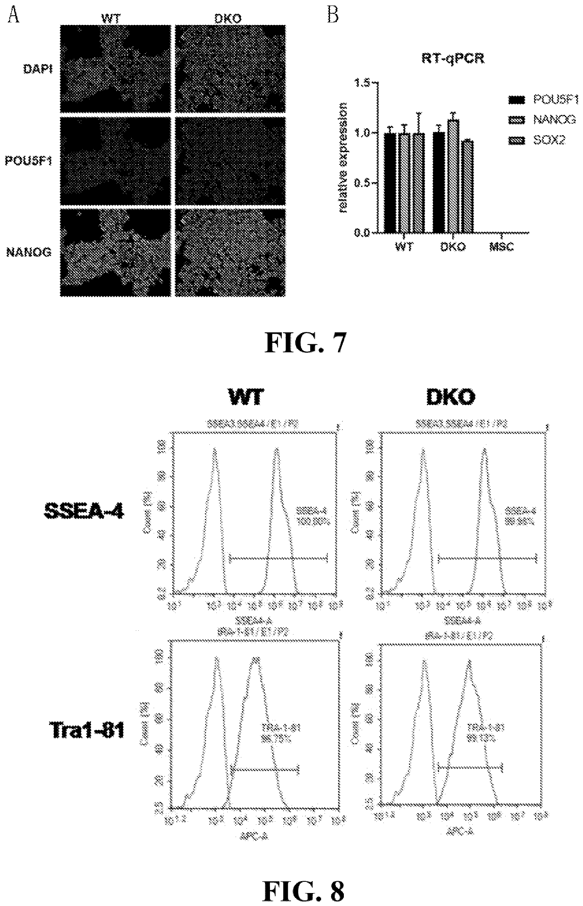

Immunofluorescence detection shows that WT and DKO cells express the stemness genes POU5F1 and NANOG at protein level: The cells were plated in a 12-well plate, the medium of which was pipetted off after the cells grew to 60-80% density, and into which was added 4% paraformaldehyde for fixation. After the cell membrane was broken, the primary antibody of POU5F1 and NANOG was used for overnight incubation at 4°C. After washing off the primary antibodies, the secondary antibody with a fluorescent label was incubated at room temperature, and then photos were taken with a fluorescent microscope. The results are shown in Fig. 7A. RT-qPCR detection shows that the WT and DKO cells express the stemness genes POU5F1, NANOG and SOX2 at RNA level: The results are shown in Fig. 7B. (MSC is the negative control of stemness gene expression)

Flow cytometry show that the stemness genes SSEA-4 and Tra1-81 are highly expressed on both of the surface of WT and DKO cells, accounting for 100%, 99.98%, 96.75%, and 99.13% respectively. The results are shown in Fig. 8.

### 2. Differentiation ability of obtained positive clone with B2M/CIITA double alleles knocked out (DKO)

100 µL of a suspension containing 5E+5 DKO cells was injected subcutaneously into immunodeficient mice (SCID Beige). After the teratoma was larger than 1.5 cm³, it was taken out, sliced and stained.

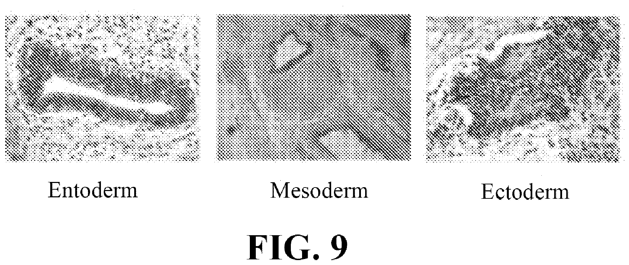

The obtained positive clone with B2M/CIITA double alleles knocked out (DKO) can form a teratoma in vivo and differentiate into cells with three germ layers: an entoderm, a mesoderm, and an ectoderm. The results are shown in Fig. 9.

### 3. Verification of immune function of DKO cells

The killing experiments of T cells and NK cells were carried out with the xCELLigence RTCA Instrument. The same number of WT and DKO cell lines were suspended in the Essential 8 medium containing IL-2, and inoculated into a 96-well E-plate coated with matrigel, into which were added activated T cells or NK cells for killing detection. The RTCA detection data were analyzed by the xCELLigence software to calculate the killing ratio and escape function.

| **Name** | **Cat No.** | **Specification** | **Manufacturer** |
|---|---|---|---|
| PE anti-human CD4 | 980804 | 500 µL/tube | Biolegend |
| APC anti-human CD8 | 980904 | 500 µL/tube | Biolegend |
| FITC anti-human CD3 | 300440 | 500 tests | Biolegend |
| APC anti-human CD16 | 301012 | 100 tests | Biolegend |
| PE anti-human CD56 (NCAM) | 318306 | 100 tests | Biolegend |
| human IL-2 | 202-1L-050/CF | 50 µg | R&D |
| Y-27632 2HCl | S1049 | 5 mg | Selleck |
| Matrigel | 354277 | 5 ml | Gibco |
| Essential 8^{™} medium | A1517001 | 500 ml | Gibco |
| E-Plate VIEW 96 PET | 300601030 | 6 pieces/box | Agilent |

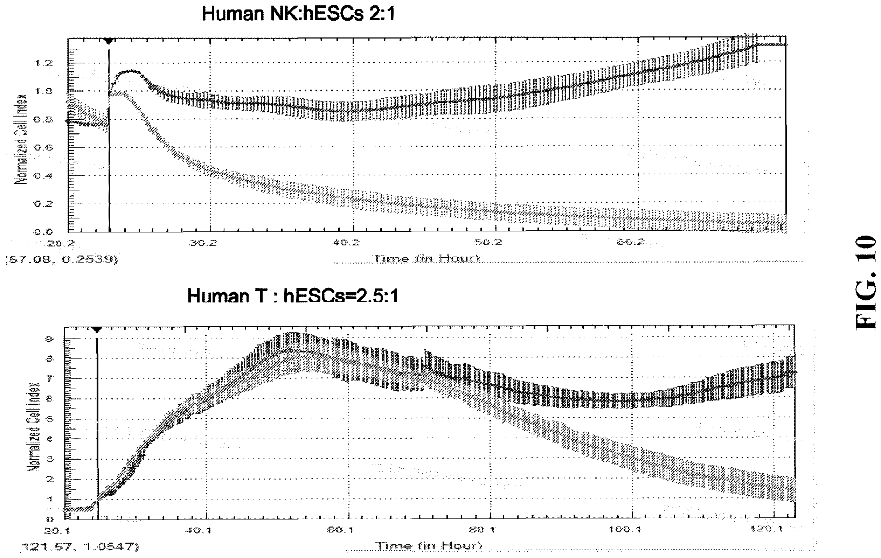

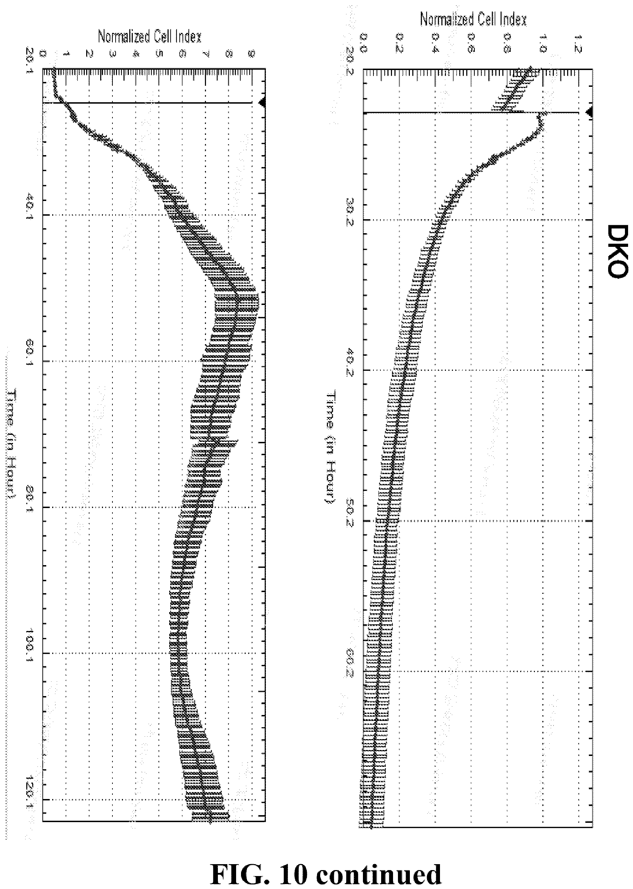

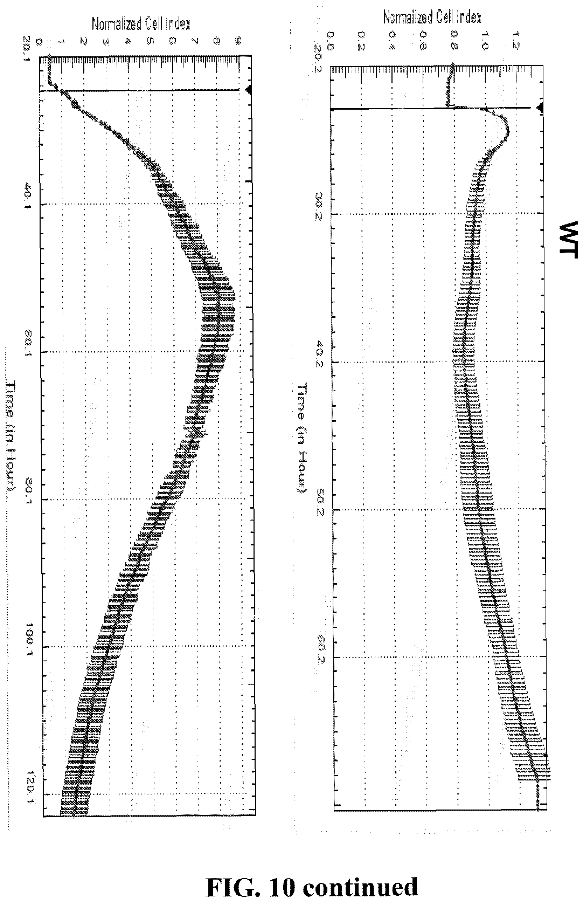

As shown in the RTCA data of Fig. 10, the WT cells escape the killing by NK cells because of the expression of HLA-I, but will be killed by T cells. The DKO cells can escape the killing by T cells, while being more sensitive to the killing by NK cells.

### Example 3. Construction and verification of immune function of DKO + CD47 cell line

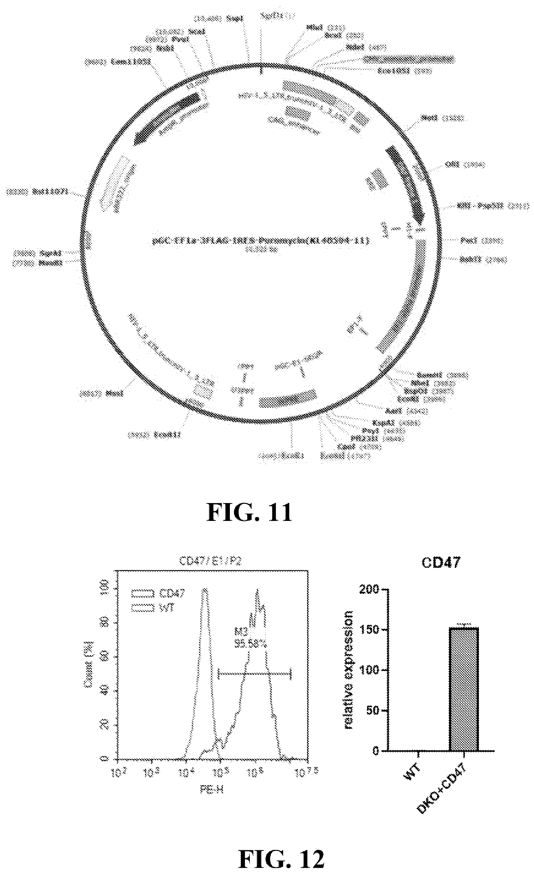

CD47 (NM_198793) was overexpressed in the DKO cells obtained in Example 1 using a lentiviral vector. The amino acid sequence of CD47 was shown in SEQ ID NO.29. The cDNA of the overexpressed sequence (SEQ ID No. 30) was constructed in a lentiviral plasmid initiated by EF1a with a puromycin screening marker (pGC-EF1a), and the structure of the pGC-EF1a plasmid is shown in Fig. 11. The plasmid was digested with *BamH*I / *Nhe*I, and after successful connection, Sanger sequencing was used to verify the correctness of the inserted sequence and viral packaging was performed. The DKO human pluripotent stem cells constructed in Example 2 were transfected, and the liquid was replaced after 24 hours. After 48 hours, it was replaced with a medium with puromycin for screening. The results of the constructed stably transfected cell strain DKO+CD47 are shown in Fig. 12. After confirming that the expression was correct, cell amplification and subsequent functional detection were carried out.

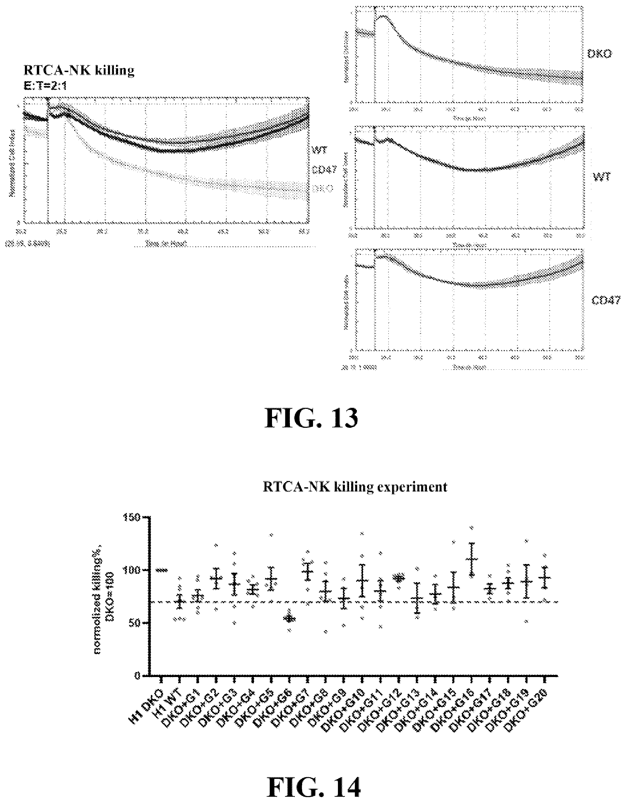

Referring to Example 2, RTCA was used to detect whether the overexpressed DKO+CD47 cell line can escape the killing by NK cells successfully while escaping the killing by T cells. As shown in Fig. 13, NK cells can effectively kill DKO cells, and WT and DKO+CD47 overexpressed cells can escape the killing by NK cells.

### Example 4. Screening of universal cells expressing fusion protein comprising functional domains of CD47 and CD24

Construction strategy and screening of fusion protein of the present invention:
CD24 is a precursor protein, which comprises a signal peptide region and a glycosyl phosphatidyl inositol (GPI) membrane anchoring sequence. After cleavage, it becomes a mature and functional CD24 protein (Chen et al., 2014; Pirruccello and LeBien, 1986). CD24 is a glycoprotein with multiple potential O- and N- glycosylation sites, which can be sialylated, thus binding to the sialic acid recognition receptor Siglec 10 (sialic acid binding Ig-like lectin 10) and interacting with macrophages to inhibit phagocytosis (Barkal et al., 2019).
CD47 is a transmembrane protein with an extracellular N- terminal IgV domain, five transmembrane domains and an intracellular C- terminal (Logtenberg et al., 2020). The N-terminal IgV domain can interact with the immunosuppressant receptor SIRPA (signal regulatory protein α), so as to inhibit immune responses (Jaiswal et al., 2009).

The strategy of the present invention is to combine known functional domains of CD24 and CD47 to form a variety of fusion proteins. Each fusion protein must comprise at least one membrane anchoring or transmembrane sequence selected from CD24 or CD47; meanwhile, each fusion protein must comprise at least one Siglec 10 or SIRPα receptor recognition sequence selected from CD24 or CD47. Each domain is linked by a flexible protein to finally form a fusion protein XSG01-XSG20. According to the method of Example 3, the fusion protein XSG001-XSG020 was overexpressed in the H1 DKO cells prepared in Example 2 by lentivirus infection, and a cell strain DKO+G1-G20 was formed, in which all fusion proteins were stably transfected.

The following are the construction strategies and specific sequences of 6 fusion proteins:
1. the fusion protein XSG006, wherein the fusion protein constructed by the functional domains of CD47 and CD24 is a fusion protein in which the mature peptide sequence of CD24 is connected to the transmembrane domain of CD47, and its amino acid sequence is as shown in SEQ ID NO.1.
2. the fusion protein XSG007, wherein the fusion protein constructed by the functional domains of CD47 and CD24 is a fusion protein in which the SIRPα binding domain of CD47 is connected to the membrane anchoring sequence of CD24, and its amino acid sequence is as shown in SEQ ID NO.35.
3. the fusion protein XSG009, wherein the fusion protein constructed by the functional domains of CD47 and CD24 is a fusion protein in which the SIRPα binding domain of CD47 is inserted into the linking site of the extracellular sequence and the membrane anchoring sequence of CD24, and its amino acid sequence is as shown in SEQ ID NO.36.
4. the fusion protein XSG010, wherein the fusion protein constructed by the functional domains of CD47 and CD24 is a fusion protein in which CD47 is linked after the extracellular sequence of CD24, and its amino acid sequence is as shown in SEQ ID NO.37.
5. the fusion protein XSG011, wherein the fusion protein constructed by the functional domains of CD47 and CD24 is a fusion protein in which the mature peptide of CD24 is linked after the SIRPα binding domain of CD47, and its amino acid sequence is as shown in SEQ ID NO.38.
6. the fusion protein XSG012, wherein the fusion protein constructed by the functional domains of CD47 and CD24 is a fusion protein in which the extracellular mature peptide of CD24 is inserted into the linking site of the SIRPα binding domain and the transmembrane domain of CD47, and its amino acid sequence is as shown in SEQ ID NO.39.

**Table 1. Amino acid sequences of some fusion proteins**

| **Sequence No** | **Amino acid sequence** | **Fusion protein name** |
|---|---|---|
| SEQ ID NO.1 | MGRAMVARLGLGLLLLALLLPTQIYSSETTTGTSSNSSQST SNSGLAPNPTNATTKAAGGGGGSGGGGSGGGGSNILIVIF PIFAILLFWGQFGIKTLKYRSGGMDEKTIALLVAGLVITVIV IVGAILFVPGEYSLKNATGLGLIVTSTGILILLHYYVFSTAIG LTSFVIAILVIQVIAYILAVVGLSLCIAACIPMHGPLLISGLSI LALAQLLGLVYMKFV | XSG006 |
| SEQ ID NO.35 | MWPLVAALLLGSACCGSAQLLFNKTKSVEFTFCNDTVVIP CFVTNMEAQNTTEVYVKWKFKGRDIYTFDGALNKSTVPT DFSSAKIEVSQLLKGDASLKMDKSDAVSHTGNYTCEVTEL TREGETIIELKYRVVSWFSPNEGGGGSGGGGSGGGGSTN ATTKAAGGALQSTASLFVVSLSLLHLYS | XSG007 |
| SEQ ID NO.36 | MGRAMVARLGLGLLLLALLLPTQIYSSETTTGTSSNSSQST SNSGLAPNPGGGGSGGGGSGGGGSQLLFNKTKSVEFTFC NDTVVIPCFVTNMEAQNTTEVYVKWKFKGRDIYTFDGAL NKSTVPTDFSSAKIEVSQLLKGDASLKMDKSDAVSHTGNY TCEVTELTREGETIIELKYRVVSWFSPNEGGGGSGGGGSG GGGSTNATTKAAGGALOSTASLFVVSLSLLHLYS | XSG009 |
| SEQ ID NO.37 | MGRAMVARLGLGLLLLALLLPTQIYSSETTTGTSSNSSQST SNSGLAPNPGGGGSGGGGSGGGGSQLLFNKTKSVEFTFCN DTVVIPCFVTNMEAQNTTEVYVKWKFKGRDIYTFDGALN KSTVPTDFSSAKIEVSQLLKGDASLKMDKSDAVSHTGNYT CEVTELTREGETIIELKYRVVSWFSPNENILIVIFPIFAILLFW GQFGIKTLKYRSGGMDEKTIALLVAGLVITVIVIVGAILFVP GEYSLKNATGLGLIVTSTGILILLHYYVFSTAIGLTSFVIAIL VIQVIAYILAVVGLSLCIAACIPMHGPLLISGLSILALAQLLG LVYMKFV | XSG010 |
| SEQ ID NO.38 | MWPLVAALLLGSACCGSAQLLFNKTKSVEFTFCNDTVVIP CFVTNMEAQNTTEVYVKWKFKGRDIYTFDGALNKSTVPT DFSSAKIEVSQLLKGDASLKMDKSDAVSHTGNYTCEVTEL TREGETIIELKYRVVSWFSPNEGGGGSGGGGSGGGGSSE TTTGTSSNSSQSTSNSGLAPNPTNATTKAAGGALQSTASLF VVSLSLLHLYS | XSG011 |
| SEQ ID NO.39 | MWPLVAALLLGSACCGSAQLLFNKTKSVEFTFCNDTVVIP CFVTNMEAQNTTEVYVKWKFKGRDIYTFDGALNKSTVPT DFSSAKIEVSQLLKGDASLKMDKSDAVSHTGNYTCEVTEL TREGETIIELKYRVVSWFSPNEGGGGSGGGGSGGGGSSE TTTGTSSNSSQSTSNSGLAPNPGGGGSGGGGSGGGGSSNI LIVIFPIFAILLFWGQFGIKTLKYRSGGMDEKTIALLVAGLV ITVIVIVGAILFVPGEYSLKNATGLGLIVTSTGILILLHYYVF STAIGLTSFVIAILVIQVIAYILAVVGLSLCIAACIPMHGPLLI SGLSILALAQLLGLVYMKFV | XSG012 |

The cell strains DKO+G1-G20 were subjected to the NK cell killing function detection as shown in Example 2, respectively, in order to screen out the fusion protein with the best degree of NK cell escape. The results are shown in Figure 14, and only DKO+G6 has a lower killing ratio than the average killing ratio of the positive control H1 WT for many times, making it the optimal cell for escaping NK killing.

### Example 5. Construction and verification of immune function of DKO + G6 cell line

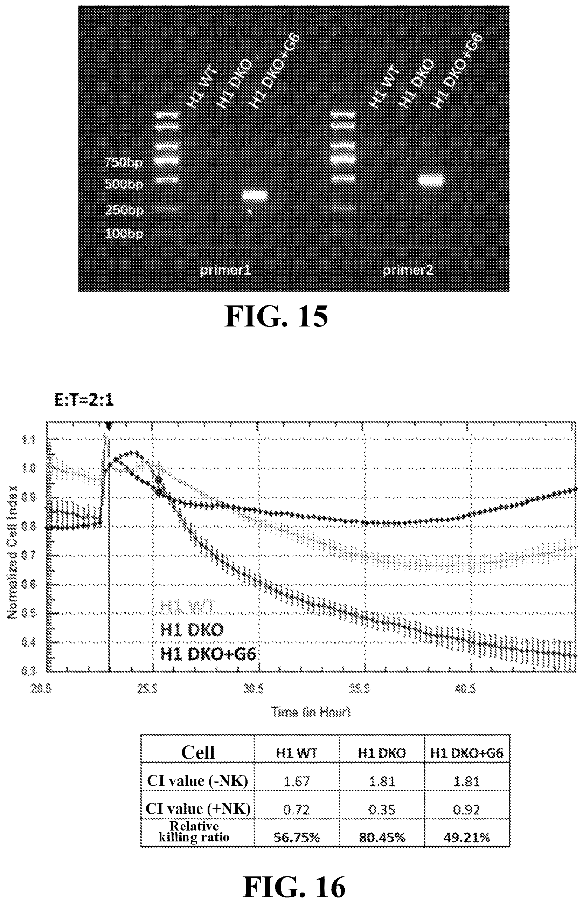

The nucleic acid sequence encoding XSG006 (the amino acid sequence of XSG006 is shown in SEQ ID NO: 1) was directly synthesized, and constructed in a lentiviral plasmid initiated by EF1a with a puromycin screening marker (pGC-EF1a), and the structure of the pGC-EF1a plasmid is shown in Fig. 11. The plasmid was digested with *BamH*I / *Nhe*I, and after successful connection, Sanger sequencing was used to verify the correctness of the inserted sequence and viral packaging was performed. The DKO human pluripotent stem cells obtained in Example 2 were transfected, and then the medium was replaced with a medium with puromycin for screening. XSG006 was a completely exogenous sequence. The insertion of the sequence in the genome of the constructed DKO+G6 cells was detected by PCR, and DKO cells were negative control. The results are shown in Fig. 15. After confirming that the expression was correct, cell amplification and subsequent functional detection were carried out.
XSG006 F1: CCAGATCTACAGCAGCGAGA
XSG006 R1: GTTCTTCAGGCTGTACTCGC
XSG006 F+XSG006 R=352bp
XSG006 F2: CCAGATCTACAGCAGCGAGA
XSG006 R2: CCAGGATGTAGGCGATCACC
XSG006 F+XSG006 R=488bp

Referring to Example 2, in the NK cell killing experiment detected by RTCA, the DKO cells constructed in Example 2 are completely killed by NK cells, and H1WT and DKO+G6 escape successfully. DKO+G6 has a degree of escape which is slightly better than that of H1 WT. The results are shown in Fig. 16.

### Example 6. Comparison of NK escape degree of several cell lines by NK cell killing experiment

Cell lines involved in this example: DKO+CD47 and DKO+CD24 in Example 3 and DKO+CD47 in the prior art (WO2020018615A2) (hereinafter referred to as "CD47 prior art"), wherein CD47 of the DKO+CD47 cell line in the prior art has an amino acid sequence shown in SEQ ID NO.40, and promotes immune escaping by interacting with a signal regulatory protein α (SIRPα) on the surface of immune cells. The construction of the "CD47 prior art" cell line is completed according to the records in WO2020018615A2. Overexpression of CD47 in cells with B2M and CIITA double knocked out (DKO) can successfully escape killing by T and NK cells. (PMID: 32433947/PMID: 30778232).

CD24 promotes immune escaping by interacting with sialic acid binding Ig-like lectin 10 (Siglec-10), an inhibitory receptor on the surface of immune cells (PMID: 31367043). CD24 of the cell line DKO+CD24 in this example has an amino acid sequence shown in SEQ ID NO.41. The construction method of the cell line refers to Example 3.

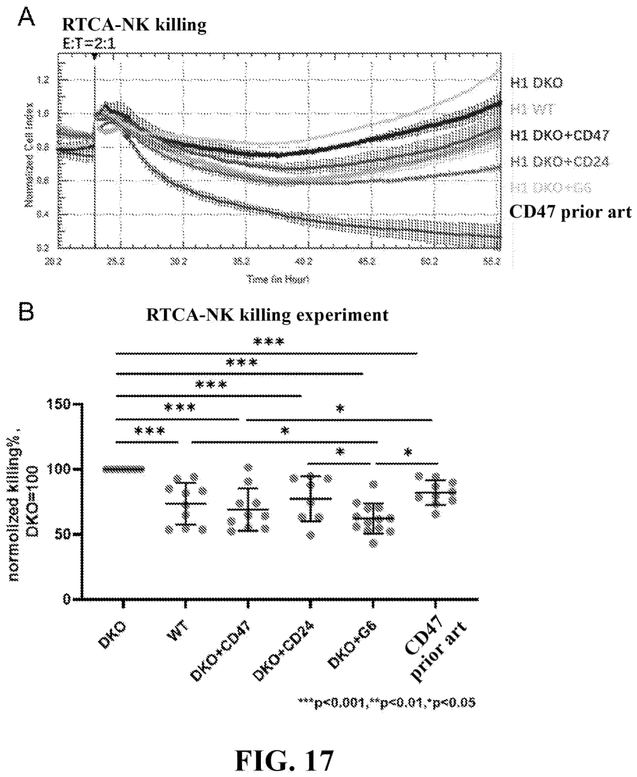

Meanwhile, the repeated experiments were normalized into each DKO killing, and the summary of the killing results shows that the killing ratio of DKO+G6 is the smallest, which is significantly different from that of WT, DKO+CD24 and "CD47 prior art". The results are shown in Fig. 17.

### Example 7. Comparison of NK escape degree of the DKO+G6 and DKO+CD47+CD24 cell lines by NK cell killing experiment

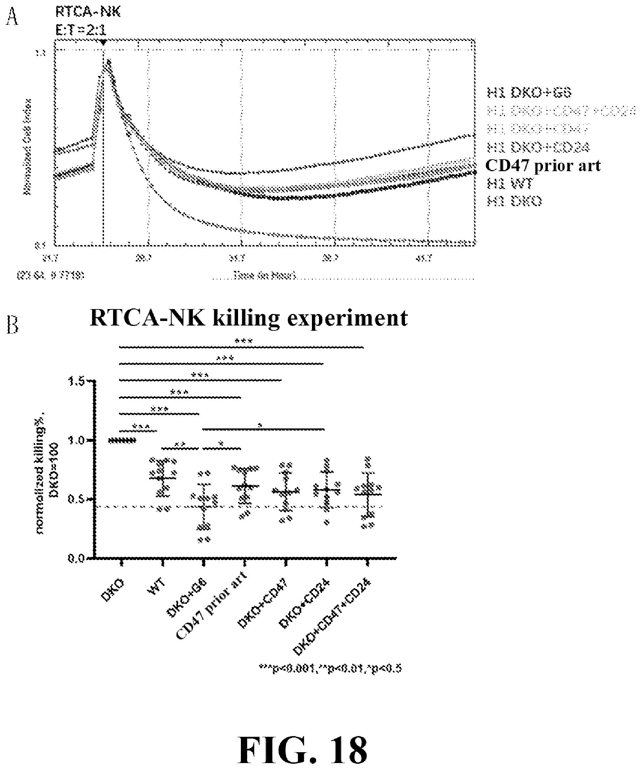

The DKO+CD47+CD24 cell line was obtained by further overexpressing CD24 by lentivirus transfection on the basis of Example 3. For lentivirus construction and overexpression operation, refer to Example 3. The amino acid sequence of CD24 is MGRAMVARLGLGLLLLALLLPTQIYSSETTTGTSSNSSQSTSNSGLAPNPTNATTKAAGG ALQSTASLFVVSLSLLHLYS. Comparing DKO+G6 with DKO+CD47+CD24 through the NK cell killing experiment, it is found that DKO+G6 cells have a smaller killing ratio and a better escaping effect. The results are shown in Fig. 18.

### Example 8. Verification of immune escaping effect of neurocytes differentiated from DKO+G6 in mice with humanized immune system

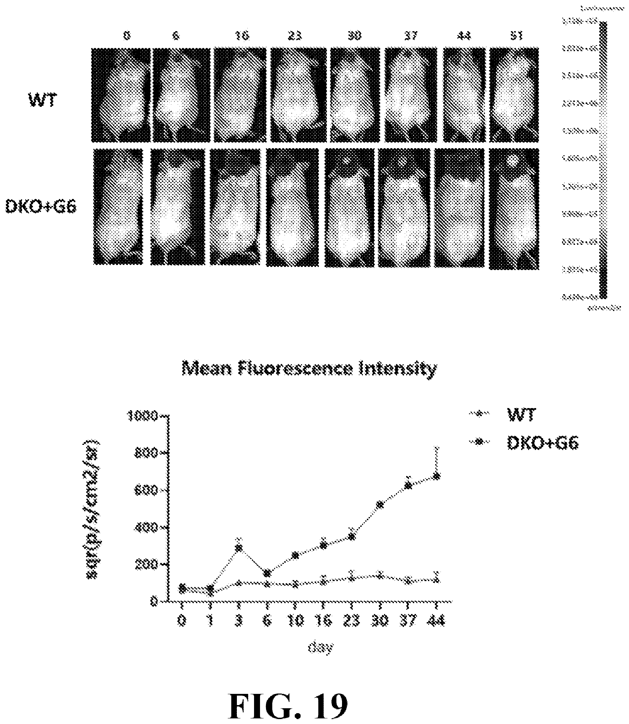

After passage, the WT and DKO+G6 cells were plated in a culture bottle that had been precoated with Matrigel, and after 24 h of culture, they were switched to a pre-differentiation medium to induce the cells to differentiate into midbrain cells.(Nolbrant S, Heuer A, Parmar M, Kirkeby A. Generation of high-purity human ventral midbrain dopaminergic progenitors for in vitro maturation and intracerebral transplantation. Nat Protoc. 2017 Sep;12(9):1962-1979. doi: 10.1038/nprot.2017.078. Epub 2017 Aug 31. PMID: 28858290); after differentiation for 9 d, high-purity midbrain cells can be obtained. Then, a large number of neural progenitor cells with high purity could be obtained by adding a neural progenitor cell medium to amplify the obtained midbrain cells; finally, the neural progenitor cells were further differentiated into neurocytes by adding a neural precursor cell medium. The differentiated WT and DKO+G6 cells were infected by lentivirus carrying luciferase (luc), and then the cells expressing luc were transplanted into mice with humanized immune system with CD34⁺HSC reconstructed. The survival of the cells in mice can be indicated by intraperitoneal injection of D-fluorescein (A025011, Shanghai Yisheng Biotechnology Co., Ltd.), a luminescent substrate of luc, and detection of the fluorescence intensity of the cells by iVIS spectrum (PerkinElmer). After continuous detection of the fluorescence at the transplantation site for 51 days, the fluorescence value of the neurocytes differentiated from WT tends to the background value, while the continuously rising fluorescence value of the neurocytes differentiated from DKO+G6 can be detected after transplantation, indicating that neurocytes differentiated from DKO+G6 can effectively escape the attack of immune system in vivo compared with the neurocytes differentiated from WT. The results are shown in Fig. 19.

The embodiments of the present invention have been described above. However, the present invention is not limited to the above-mentioned embodiments. Any modifications, equivalent substitutions, improvements, etc., made within the spirit and principles of the present invention should be comprised in the protection scope of the present invention.

## Claims

1. A universal cell, relative to a wild-type cell, comprising:
1) reduced expression or no expression of MHC-I and/or MHC-II human leukocyte antigens; and
2) expression of a fusion protein comprising immune inhibitory checkpoints;
wherein the cell can escape T cell attack and killing by NK cells;
preferably, the fusion protein comprising the immune inhibitory checkpoints comprises two or more of PD-L1, CTLA4-Ig, CD47, and CD24; preferably, a fusion protein comprising two of the immune inhibitory checkpoints is expressed; more preferably, a fusion protein comprising functional domains of CD47 and/or CD24 is expressed.

2. The universal cell according to claim 1, wherein the cell comprises reduced expression or no expression of MHC-I and MHC-II human leukocyte antigens.

3. The universal cell according to claim 1 or 2, wherein gene editing tools are used in the cell to target one or more genes encoding one or more transcriptional regulatory factors of MHC-I, or one or more genes encoding one or more transcriptional regulatory factors of MHC-II, so as to achieve reduced expression or no expression of MHC-I and/or MHC-II genes;
preferably, the transcriptional regulatory factor of MHC-I is selected from one or more of B2M, TAP1, TAP2, TAP-related glycoproteins (Tapasin) or NLRC5; the transcriptional regulatory factor of MHC-II is selected from one or more of CIITA, RFXANK, RFX5 and RFXAP;
further preferably, the transcriptional regulatory factors are B2M and CIITA.

4. The universal cell according to claim 3, wherein the cell further comprises a genetic modification targeting a CIITA gene by a rare cutting endonuclease that selectively inactivates the CIITA gene.

5. The universal cell according to any one of claims 1-4, wherein the cell further comprises a genetic modification targeting a B2M gene by a rare cutting endonuclease that selectively inactivates the B2M gene.

6. The universal cell according to claim 4 or 5, wherein the rare cutting endonuclease is selected from CAS proteins, TALE-nucleases, zinc finger nucleases, large nucleases and homing nucleases.

7. The universal cell according to claim 6, wherein the genetic modification targeting the CIITA gene or the B2M gene by the rare cutting endonuclease comprises a CAS protein or a polynucleotide encoding a CAS protein, and at least one guide ribonucleic acid sequence for specifically targeting the CIITA gene or the B2M gene.

8. The universal cell according to claim 7, wherein a CRISPR/CAS9 system is used to directly knock out the exon segments of B2M and CIITA at both ends, respectively, wherein the target sequences of the guide ribonucleic acid sequence gRNA for the B2M gene are SEQ ID NO: 2 and 3, and the target sequences of the guide ribonucleic acid sequence gRNA for the CIITA gene are SEQ ID NO: 4 and 5.

9. The universal cell according to claim 1 or 2, wherein gene expression modifying molecules for one or more genes encoding one or more transcriptional regulatory factors of MHC-I, or gene expression modifying molecules for one or more genes encoding one or more transcriptional regulatory factors of MHC-II are introduced into the cells, so as to achieve reduced expression or no expression of MHC-I and/or MHC-II genes, wherein the gene expression modifying molecules comprise one selected from siRNA, shRNA, microRNA, antisense RNA and another RNA-mediated inhibitory molecule.

10. The universal cell according to any one of claims 1-9, wherein the functional domain of CD47 in the fusion protein comprising the functional domains of CD47 and/or CD24 is a transmembrane domain of CD47; preferably, the amino acid sequences of the transmembrane domains of CD47 are as shown in any one of SEQ ID Nos: 6-10.

11. The universal cell according to any one of claims 1-10, wherein the functional domain of CD24 in the fusion protein comprising the functional domains of CD47 and/or CD24 is a signal peptide sequence of CD24, a mature peptide of CD24, an extracellular peptide of CD24, a membrane anchoring sequence of CD24, and an extracellular mature peptide of CD24, preferably, the amino acid sequences of the functional domains of CD24 are as shown in any one of SEQ ID NOs: 11-16.

12. The universal cell according to claim 11, wherein the fusion protein constructed by the functional domains of CD47 and CD24 is a fusion protein in which a SIRPα binding domain of CD47 is connected to the membrane anchoring sequence of CD24.

13. The universal cell according to claim 11, wherein the fusion protein constructed by the functional domains of CD47 and CD24 is a fusion protein in which the SIRPα binding domain of CD47 is inserted into the linking site of the extracellular sequence and the membrane anchoring sequence of CD24.

14. The universal cell according to claim 11, wherein the fusion protein constructed by the functional domains of CD47 and CD24 is a fusion protein in which the SIRPα binding domain of CD47 is linked after the extracellular sequence of CD24.

15. The universal cell according to claim 11, wherein the fusion protein constructed by the functional domains of CD47 and CD24 is a fusion protein in which the mature peptide of CD24 is linked after the SIRPα binding domain of CD47.

16. The universal cell according to claim 11, wherein the fusion protein constructed by the functional domains of CD47 and CD24 is a fusion protein in which the extracellular mature peptide of CD24 is inserted into the linking site of the SIRPα binding domain and the transmembrane domain of CD47.

17. The universal cell according to claim 11, wherein the fusion protein constructed by the functional domains of CD47 and CD24 is a fusion protein in which the mature peptide sequence of CD24 is connected to the transmembrane domain of CD47;
preferably, the amino acid sequence of the fusion protein constructed by the functional domains of CD47 and CD24 has more than 70% homology with the sequence shown in SEQ ID NO: 1, for example more than 80% homology, and for example more than 90%, more than 95%, or more than 98% homology;
further preferably, the amino acid sequence of the fusion protein constructed by the functional domains of CD47 and CD24 is as shown in SEQ ID NO:1.

18. The universal cell according to any one of claims 1-17, wherein the cell further comprises modifications to increase the expression of one or more of the following polypeptides: CD35, CD27, DUX4, CD26, CD55, CD59, CD200, HLA-C, HLA-E, the HLA-E heavy chain, HLA-G, PD-L1, IDO1, IDO2, TDO, CTLA4-IgG , the Cl-inhibitor, IL-10, CD46, CD55, CD59, CCL21, Mfge8, SerpinB9 and IL-35.

19. The universal cell according to any one of claims 1-18, wherein the cell is an embryonic stem cell or a pluripotent stem cell; preferably a stem cell with low immunogenicity; further preferably a human stem cell or a human somatic cell.

20. A method for preparing the universal cell according to any one of claims 1-19, comprising the following steps:
1) knocking out one or more genes of one or more transcriptional regulatory factors of MHC-I of stem cells; and/or
2) knocking out one or more genes of one or more transcriptional regulatory factors of MHC-II of the stem cells; and
3) introducing a nucleic acid sequence encoding a protein comprising immune inhibitory checkpoints into the stem cells;
preferably, the protein comprising immune inhibitory checkpoints comprises one or more of PD-L1, CTLA4-Ig, CD47, and CD24; preferably, a nucleic acid sequence encoding a fusion protein comprising two of the immune inhibitory checkpoints are introduced; more preferably, a nucleic acid sequence encoding a fusion protein constructed by comprising functional domains of CD47 and/or CD24 is introduced.

21. The method for preparing the universal cell according to claim 20, wherein the transcriptional regulatory factor of MHC-I is selected from one or more of B2M, TAP1, TAP2, TAP-related glycoproteins (Tapasin) or NLRC5; the transcriptional regulatory factor of MHC-II is selected from one or more of CIITA, RFXANK, RFX5 and RFXAP; preferably, the transcriptional regulatory factors are selected from B2M and CIITA.

22. The preparation method according to claim 20 or 21, wherein the knocking out in step 1) or 2) is a genetic modification targeting a CIITA gene or a B2M gene by a rare cutting endonuclease that selectively inactivates the CIITA gene or the B2M gene;
preferably, the rare cutting endonuclease is selected from CAS proteins, the TALE-nucleases, zinc finger nucleases, large nucleases and homing nucleases;
further preferably, the genetic modification targeting the CIITA gene or the B2M gene by the rare cutting endonuclease comprises a CAS protein or a polynucleotide encoding a CAS protein, and at least one guide ribonucleic acid sequence for specifically targeting the CIITA gene or the B2M;
still further preferably, a CRISPR system is used in steps 1) and 2) to directly knock out the exon segments of B2M and CIITA at both ends, respectively, wherein the target sequences of gRNA for the B2M gene are SEQ ID NO: 2 and 3, and the target sequences of gRNA for the CIITA gene are SEQ ID NO: 4 and 5.

23. The preparation method according to claim 20 or 21, wherein the knocking out in step 1) or 2) is introducing gene expression modifying molecules for one or more genes encoding one or more transcriptional regulatory factors of MHC-I, or for one or more genes encoding one or more transcriptional regulatory factors of MHC-II, so as to achieve reduced expression or no expression of MHC-I and/or MHC-II genes, wherein the gene expression modifying molecules comprise one selected from siRNA, shRNA, microRNA, antisense RNA and another RNA-mediated inhibitory molecule.

24. The preparation method according to any one of claims 20-23, wherein in the step 3), an expression vector is used to introduce a nucleic acid sequence encoding a fusion protein comprising functional domains of CD47 and/or CD24 into stem cells;
preferably, the expression vector used in the step 3) is a viral vector;
further preferably, the viral vector is a lentivirus.

25. The preparation method according to any one of claims 20-24, wherein in the step 3), the nucleic acid sequence encoding the fusion protein comprising the functional domains of CD47 and/or CD24 is introduced into a selected site of the stem cells; preferably, the selected site of the stem cell is a safe harbor gene site.

26. The preparation method according to any one of claims 20-25, wherein the functional domain of CD47 in the fusion protein comprising the functional domains of CD47 and/or CD24 is a transmembrane domain of CD47; preferably, the amino acid sequences of the transmembrane domains of CD47 are as shown in any one of SEQ ID Nos: 6-10.

27. The preparation method according to any one of claims 20-26, wherein the functional domain of CD24 in the fusion protein comprising the functional domains of CD47 and/or CD24 is a signal peptide sequence of CD24, a mature peptide of CD24, an extracellular peptide of CD24, a membrane anchoring sequence of CD24, and an extracellular mature peptide of CD24, preferably, the amino acid sequences of the functional domains of CD24 are as shown in any one of SEQ ID NOs:11-16.

28. The preparation method according to claim 27, wherein the fusion protein constructed by the functional domains of CD47 and CD24 is a fusion protein in which a SIRPα binding domain of CD47 is connected to the membrane anchoring sequence of CD24.

29. The preparation method according to claim 27, wherein the fusion protein constructed by the functional domains of CD47 and CD24 is a fusion protein in which the SIRPα binding domain of CD47 is inserted into the linking site of the extracellular sequence and the membrane anchoring sequence of CD24.

30. The preparation method according to claim 27, wherein the fusion protein constructed by the functional domains of CD47 and CD24 is a fusion protein in which the SIRPα binding domain of CD47 is linked after the extracellular sequence of CD24.

31. The preparation method according to claim 27, wherein the fusion protein constructed by the functional domains of CD47 and CD24 is a fusion protein in which the mature peptide of CD24 is linked after the SIRPα binding domain of CD47.

32. The preparation method according to claim 27, wherein the fusion protein constructed by the functional domains of CD47 and CD24 is a fusion protein in which the extracellular mature peptide of CD24 is inserted into the linking site of the SIRPα binding domain and the transmembrane domain of CD47.

33. The preparation method according to claim 27, wherein the fusion protein constructed by the functional domains of CD47 and CD24 is a fusion protein in which the transmembrane domain of CD47 is connected to the mature peptide sequence of CD24; preferably, the fusion protein constructed by the functional domains of CD47 and CD24 has more than at least 70% homology with the sequence shown in SEQ ID NO: 1, for example more than 80% homology, and for example more than 90%, more than 95%, or more than 98% homology; further preferably, the amino acid sequence of the fusion protein constructed by the functional domains of CD47 and CD24 is as shown in SEQ ID NO:1.

34. The preparation method according to any one of claims 20-33, wherein the universal stem cell further comprises a second expression vector, comprising a polynucleotide sequence encoding one selected from CD35, CD27, DUX4, CD26, CD55, CD59, CD200, HLA-C, HLA-E, the HLA-E heavy chain, HLA-G, PD-L1, IDO1, IDO2, TDO, CTLA4-IgG, the Cl-inhibitor, IL-10, CD46, CD55, CD59, CCL21, Mfge8, SerpinB9 and IL-35.

35. The preparation method according to claim 34, wherein the second expression vector is an inducible expression vector; preferably, the second expression vector is a viral vector.

36. A method for preparing differentiated universal cells, comprising culturing the universal cells prepared according to the method according to any one of claims 20-35 under differentiation conditions, thereby preparing differentiated cells with low immunogenicity.

37. The method according to claim 36, wherein the differentiation conditions are suitable for differentiating cells into a cell type selected from cardiomyocytes, neurocytes, glial cells, endothelial cells, T cells, NK cells, NKT cells, macrophages, hematopoietic progenitor cells, mesenchymal cells, pancreatic islet cells, chondrocytes, retinal pigment epithelial cells, kidney cells, hepatocytes, thyroid cells, skin cells, blood cells and epithelial cells.

38. A method for treating a patient in need of cell therapy, comprising administering the differentiated cell population with low immunogenicity prepared according to the method according to claim 36 or 37.

39. A composition, comprising the universal cell according to any one of claims 1-29; preferably, the composition further comprises one or more therapeutic agents; preferably, the therapeutic agent comprises peptides, cytokines, checkpoint inhibitors, mitogens, growth factors, small RNAs, double stranded RNAs (dsRNAs), mononuclear blood cells, feeder cells, feeder cell components or replacement factors thereof, vectors comprising one or more polynucleic acids of interest, antibodies, etc.

40. A fusion protein, comprising functional domains of CD47 and/or CD24.

41. The fusion protein according to claim 40, wherein the functional domain of CD47 in the fusion protein comprising the functional domains of CD47 and/or CD24 is a transmembrane domain of CD47; preferably, the amino acid sequences of the transmembrane domains of CD47 are as shown in any one of SEQ ID Nos: 6-10.

42. The fusion protein according to claim 40, wherein the functional domain of CD24 in the fusion protein comprising the functional domains of CD47 and/or CD24 is a signal peptide sequence of CD24, a mature peptide of CD24, an extracellular peptide of CD24, a membrane anchoring sequence of CD24, and an extracellular mature peptide of CD24, preferably, the amino acid sequences of the functional domains of CD24 are as shown in any one of SEQ ID NOs: 11-16.

43. The fusion protein according to claim 42, wherein the fusion protein constructed by the functional domains of CD47 and CD24 is a fusion protein in which a SIRPα binding domain of CD47 is connected to the membrane anchoring sequence of CD24.

44. The fusion protein according to claim 42, wherein the fusion protein constructed by the functional domains of CD47 and CD24 is a fusion protein in which the SIRPα binding domain of CD47 is inserted into the linking site of the extracellular sequence and the membrane anchoring sequence of CD24.

45. The fusion protein according to claim 42, wherein the fusion protein constructed by the functional domains of CD47 and CD24 is a fusion protein in which the SIRPα binding domain of CD47 is linked after the extracellular sequence of CD24.

46. The fusion protein according to claim 42, wherein the fusion protein constructed by the functional domains of CD47 and CD24 is a fusion protein in which the mature peptide of CD24 is linked after the SIRPα binding domain of CD47.

47. The fusion protein according to claim 42, wherein the fusion protein constructed by the functional domains of CD47 and CD24 is a fusion protein in which the extracellular mature peptide of CD24 is inserted into the linking site of the SIRPα binding domain and the transmembrane domain of CD47.

48. The fusion protein according to claim 42, wherein the fusion protein constructed by the functional domains of CD47 and CD24 is obtained by inserting the transmembrane domain of CD47 into the linking site of the signal peptide sequence and a precursor protein sequence of CD24; or connecting the transmembrane domain of CD47 to the signal peptide sequence and the mature peptide sequence of CD24; preferably, the amino acid sequence of the fusion protein has more than 70% homology with the sequence shown in SEQ ID NO: 1, for example more than 80% homology, and for example more than 90%, more than 95%, or more than 98% homology; further preferably, the amino acid sequence of the fusion protein constructed by the functional domains of CD47 and CD24 is as shown in SEQ ID NO:1.

49. A nucleic acid sequence encoding the fusion protein according to any one of claims 40-48, **characterized in that** the nucleic acid sequence can be obtained based on the amino acid sequence of the fusion protein according to any one of claims 40-48.

50. An expression vector or expression cassette, comprising the nucleic acid sequence according to claim 49.

51. A cell, comprising the expression of the fusion protein according to any one of claims 40-48, and reduced expression or no expression of MHC class I and/or MHC class II human leukocyte antigens.

52. A cell, comprising no expression of CIITA, the expression of the fusion protein according to any one of claims 40-48, and reduced expression or no expression of MHC class I and/or MHC class II human leukocyte antigens.

53. A cell, comprising no expression of B2M, the expression of the fusion protein according to any one of claims 40-48, and reduced expression or no expression of MHC class I and/or MHC class II human leukocyte antigens.

54. A cell, comprising no expression of CIITA and B2M, the expression of the fusion protein according to any one of claims 40-48, and reduced expression or no expression of MHC class I and/or MHC class II human leukocyte antigens.

55. A cell, comprising the expression of the fusion protein according to any one of claims 40-48 and at least one polypeptide selected from CD35, CD27, DUX4, CD26, CD55, CD59, CD200, HLA-C, HLA-E, the HLA-E heavy chain, HLA-G, PD-L1, IDO1, IDO2, TDO, CTLA4-IgG , the Cl-inhibitor, IL-10, CD46, CD55, CD59, CCL21, Mfge8, SerpinB9 and IL-35, and reduced expression or no expression of MHC class I and/or MHC class II human leukocyte antigens.

56. A cell, comprising no expression of CIITA, the expression of the fusion protein according to any one of claims 40-48 and at least one polypeptide selected from DUX4, HLA-C, HLA-E, HLA-G, PD-L1, CTLA4, the Cl-inhibitor, CD46, CD55, CD59, and IL-35, and reduced expression or no expression of MHC class I and/or MHC class II human leukocyte antigens.

57. A cell, comprising no expression of B2M, the expression of the fusion protein according to any one of claims 40-48 and at least one polypeptide selected from DUX4, HLA-C, HLA-E, HLA-G, PD-L1, CTLA4, the Cl-inhibitor, CD46, CD55, CD59, and IL-35, and reduced expression or no expression of MHC class I and/or MHC class II human leukocyte antigens.

58. A cell, comprising no expression of CIITA and B2M, the expression of the fusion protein according to any one of claims 40-489 and at least one polypeptide selected from DUX4, HLA-C, HLA-E, HLA-G, PD-L1, CTLA4, the Cl-inhibitor, CD46, CD55, CD59, and IL-35, and reduced expression or no expression of MHC class I and/or MHC class II human leukocyte antigens.

59. The cell according to any one of claims 52-58, wherein the cell is selected from stem cells, differentiated cells, pluripotent stem cells, induced pluripotent stem cells, adult stem cells, progenitor cells, somatic cells, primary T cells and chimeric antigen receptor T cells.

60. Use of the universal cell according to any one of claims 1-19, the composition according to claim 39, the fusion protein according to any one of claims 40-48, or the expression vector or expression cassette according to claim 50, and the cell according to claims 52-58 in the preparation of a product for cell therapy.

61. Use of the universal cell according to any one of claims 1-19, the composition according to claim 39, the fusion protein according to any one of claims 40-48, or the expression vector or expression cassette according to claim 50, and the cell according to claims 52-58 in the preparation of a product for organ transplantation.

62. Use of the universal cell according to any one of claims 1-19, the composition according to claim 39, the fusion protein according to any one of claims 40-48, or the expression vector or expression cassette according to claim 50, and the cell according to claims 52-58 in the construction of cell libraries of universal PSCs.

63. Use of the universal cell according to any one of claims 1-19, the composition according to claim 39, the fusion protein according to any one of claims 40-48, or the expression vector or expression cassette according to claim 50, and the cell according to claims 52-58 as gene drug carriers.
